# EUROPEAN PATENT APPLICATION

(11) **EP 1 347 043 A1**
(43) Date of publication of application: **24.09.2003**
(21) Application number: 03005361.5
(22) Date of filing: 12.03.2003
(51) Int. Cl.: C12N 9/12

(54) **Unary avian myeloblastosis virus reverse transcriptase and its use**

(30) Priority: 15.03.2002 JP 2002071841
(71) Applicant: Tosoh Corporation, Shinnanyo-shi, Yamaguchi-ken, 746-8501 (JP)
(72) Inventor: Yasukawa, Kiyoshi, Kawasaki-shi, Kanagawa-ken (JP); Ishiguro, Takahiko, Yokohama-shi, Kanagawa-ken (JP)
(74) Representative: VOSSIUS & PARTNER

(57) **Abstract**

An avian myeloblastosis virus reverse transcriptase composition which comprises a heterodimer consisting of an α subunit having a molecular weight of 68000 and a β subunit having a molecular weight of 92000 in an amount of at least 80%.

## Description

The present invention relates an avian myeloblastosis virus reverse transcriptase composition.

Avian myeloblastosis virus reverse transcriptase (hereinafter referred to as "AMVRT") is an occurring enzyme that can be isolated from avian myeloblastosis virus (AMV) carriers and is used for research in the field of molecular biology and gene amplification. An AMVRT isolate from a natural source is known to be a ternary composition comprising the α monomer consisting of one molecule of the α subunit having a molecular weight of 68000 (arising from fragmentation of the β subunit), a heterodimer (αβ heterodimer) consisting of one molecule of the α subunit and one molecule of the β subunit having a molecular weight of 92000, and a homodimer (ββ homodimer) consisting of two molecules of the β subunit.

The main catalytic activity of AMVRT is the reverse transcriptase activity, or the RNA-dependent DNA polymerase activity, and the activity of AMVRT is conventionally expressed through its RNA-dependent DNA polymerase activity measured in units by using poly(A)· oligo(dT) as the template under the definition that one unit is required to incorporate 1 nmol of deoxyribonucleotides into acid precipitable material in 10 minutes at 37°C.

In recent years, gene diagnosis based on qualitative and quantitative trace analysis of nucleic acid in test samples is widely used for early identification of infectious or other diseases and monitoring of the effects of therapies for them, screening of blood supplies and food and environment monitoring. AMVRT is used in gene amplification preceding gene diagnosis.

Gene amplification using AMVRT is exemplified by so-called NASBA (Nucleic Acid Sequence Based Amplification) disclosed in Patent No.2650159 and so-called TMA (Transcription-Mediated Amplification) disclosed in JP-A-4-500759 and TRC (Transcription-Reverse transcription Concerted reaction) reported by the present inventors in JP-A-2000-14400.

The above-mentioned amplification techniques give RNA copies homologous or complementary to the nucleic acid (target RNA) to be detected transcriptionally at relatively low and constant temperatures. A specific method comprises, for example, hybridizing a first DNA primer (having a promoter region) to a target RNA, elongating the first DNA primer into a DNA-RNA heteroduplex nucleic acid, degrading the target RNA in the heteroduplex nucleic acid, hybridizing a second DNA primer to the residual DNA, elongating the second DNA primer into a double-stranded DNA having the promoter region, and synthesizing RNA copies complementary to the target RNA from the double-stranded DNA (and thereby builds an amplification cycle comprising hybridization of the first DNA primer to the resulting RNA copies, elongation of the first DNA primer into a DNA-RNA heteroduplex nucleic acid, degradation of the target RNA in the heteroduplex nucleic acid, hybridization of the second DNA primer to the residual DNA, elongation of the second DNA primer into a double-stranded DNA having a promoter region and synthesis of RNA copies complementary to the target RNA from the double-stranded DNA). Another specific method comprises, for example, hybridizing a first DNA primer to a target RNA, elongating the first DNA primer into a DNA-RNA heteroduplex nucleic acid, degrading the target RNA in the heteroduplex nucleic acid, hybridizing a second DNA primer (having a promoter region) to the residual DNA, elongating the second DNA primer into a double-stranded DNA having the promoter region, and synthesizing RNA copies homologous to the target RNA (and thereby builds an amplification cycle comprising hybridization of the first DNA primer to the resulting RNA copies, elongation of the first DNA primer into a DNA-RNA heteroduplex nucleic acid, degradation of the target RNA in the heteroduplex nucleic acid, hybridization of the second DNA primer to the residual DNA, elongation of the second DNA primer into a double-stranded DNA having a promoter region and synthesis of RNA copies homologous to the target RNA from the double-stranded DNA). In these amplification techniques, the target RNA may be an RNA obtained from a single-stranded DNA anticipated in a sample through reverse transcription or transcription as in the above-mentioned reactions.

AMVRT has not only an RNA-dependend DNA polymerase activity but also a DNA-dependent DNA polymerase activity and a ribonuclease H activity. In the above-mentioned techniques, not only the main activity of AMVRT as an RNA-dependent DNA polymerase, but also the DNA-dependent DNA polymerase activity and the ribonuclease H activity matter. Especially, the ribonuclease H activity is crucial to omit the heating operation for split of the heteroduplex and the cooling operation for the subsequent reactions and is extremely crucial to progress amplification without RNase H replenishment.

However, as is evident from the fact that the catalytic activity of AMVRT has been expressed through its reverse transcriptase activity (the RNA-dependent DNA polymerase activity), the constituents of AMVRT have drawn attention only in terms of the reverse transcriptase activity and have never been studied in terms of the other activities. In other words, although it is known that the α monomer, the αβ heterodimer and the ββ homodimer all show the reverse transcriptase activity of AMVRT, the other activities have not been studied at all.

Under these circumstances, the present inventors' extensive studies on the constituents of AMVRT surprisingly have revealed that though the α monomer, the αβ heterodimer and the ββ homodimer all show the reverse transcriptase activity, when these constituents are used separately as a reagent in the above-mentioned amplification techniques, only the αβ heterodimer, but neither the α monomer nor the ββ homodimer, could set off the amplification reaction effectively.

The first object of the present invention is to provide AMVRT useful for the above-mentioned amplification techniques. The second object of the present invention is to provide a method of preparing the AMVRT. The third object of the present invention is to provide a reagent comprising the AMVRT for use in the above-mentioned amplification of nucleic acid.

In order to attain the first object mentioned above, the present invention defined in Claim 1 of the present application provides an AMVRT composition which comprises a heterodimer (αβ heterodimer) consisting of an α subunit having a molecular weight of 68000 and a β subunit having a molecular weight of 92000 in an amount of at least 80%. The invention defined in Claim 2 of the present application provides the AMVRT composition according to Claim 1 which is a unary composition consisting of a heterodimer (αβ heterodimer) consisting of an α subunit having a molecular weight of 68000 and a β subunit having a molecular weight of 92000.

In order to attain the second object mentioned above, the present invention defined in Claim 3 of the present application provides a method of preparing a heterodimer (αβ heterodimer) consisting of an α subunit having a molecular weight of 68000 and a β subunit having a molecular weight of 92000 from an AMVRT solution containing the αβ heterodimer, and the α subunit (α monomer) and/or the homodimer (ββ homodimer) of the β subunit, which comprises subjecting the solution to ion exchange chromatography. The invention defined in Claim 4 of the present application provides the method according to Claim 3, wherein the ion exchange chromatography uses an ion exchanger having diethylaminoethyl (DEAE) groups as ion exchange groups.

In order to attain the third object mentioned above, the present invention defined in Claim 5 of the present application provides a reagent for use in amplification of a target RNA or an RNA complementary to the target RNA comprising hybridizing a first DNA primer to the target RNA, elongating the first DNA primer into a DNA-RNA heteroduplex nucleic acid, degrading the target RNA in the DNA-RNA heteroduplex nucleic acid, hybridizing a second DNA primer to the residual DNA, elongating the second DNA primer into a double-stranded DNA, transcribing the double-stranded DNA into the target RNA or an RNA complementary to the target RNA (wherein either the first or second DNA primer has a promoter region or a region complementary to the promoter region), which comprises the AMVRT composition according to Claim 1.

Fig. 1 shows the results of the electrophoresis of fractions with even numbers between 2-60 in Example 1 on a silver-stained electrophoresis gel. In lane M is a molecular marker, and AMV 8U denotes unfractionated AMVRT.

Fig. 2 shows the results of the RT activity assay of fractions with even numbers between 10 and 50 in Example 2.

Fig. 3 shows the results of the TRC reaction in the presence of fractions with even numbers between 20 and 48 for evaluation of their activities in Example 3.

Fig. 4 shows the results of the TRC reaction using the αβ concentrates obtained in Example 4. αβ concentrate 1 denotes concentrate 1, and αβ concentrate 2 denotes concentrate 2. In the negative control run, the TRC reaction was carried out without adding RNA. The initial amount of the RNA was 10³ copies/run.

Now, the present invention will be described in detail. The AMVRT composition (αβ heterodimer) of the present invention is obtainable from AMVRT isolated from AMV carriers or commercially available AMVRT preparations from natural sources (such as CHIMERx's product under the product name of AMV Reverse Transcriptase; 40 U/µl). Currently available recombinant AMVRT, which consists of the α monomer alone, is not suitable as the starting material for preparation of the AMVRT composition of the present invention.

The AMVRT composition of the present invention is obtainable from commercially available AMVRT preparations as mentioned above by liquid chromatography. Liquid chromatography is preferably high performance liquid chromatography if a column (filler) with sufficient mechanical strength is used.

In the liquid chromatography, for example, an ether column is used. Ion exchange chromatography using, as the filler, an ion exchanger having DEAE groups as the ion exchange groups is particularly preferred. The filler matrix may be silica or a polymer without particular restrictions and preferably has a large peptide adsorption capacity. On the other hand, an ordinary stainless steel, PEEK (poly ether ether ketone) or glass column may be used to accommodate the filler without particular restrictions. An example of preferable commercially available packed columns is TSKgel DEAE-5PW (product name, Tosoh Corporation).

The starting material for the AMVRT composition of the present invention is an AMVRT solution obtained by dissolving commercially available AMVRT in an appropriate buffer. The buffer to be used for the solution may, for example, be a buffer containing 20 mM calcium phosphate (pH 7.2), Triton X-100 (0.2%), dithiothreitol (hereinafter referred to as DTT) (2mM) and glycerol (10%) without particular restrictions. DTT is added to protect AMVRT. According to the present inventors' findings, the AMVRT αβ heterodimer does not dissociate into monomers at a DTT concentration around 2 mM. Triton X-100 is added to prevent adsorption of AMVRT by the column. According to the present inventors' findings, the AMVRT αβ heterodimer does not dissociate into monomers at a Triton X-100 concentration around 0.2%.

After an AMVRT solution in the above-mentioned buffer is supplied to the column, the AMVRT retained in the column is eluted. AMVRT may be eluted, for example, by supplying a buffer to the column with gradual or stepwise increase in salt concentration. For example, when the above-mentioned buffer (hereinafter referred to as buffer A) is used to dissolve AMVRT and load AMVRT onto the filler, a buffer (hereinafter referred to as buffer B) containing 20 mM calcium phosphate (pH 7.2), Triton X-100 (0.2%), DTT (2mM), glycerol (10%) and NaCl (1M) may be mixed in certain ratios. More specifically, for example, firstly buffer A is supplied at a flow rate around 1 ml/min for about 15 minutes to wash away the unadsorbable components from the filler, then the ratio of buffer B is increased gradually to about 50% over 20 minutes and then to 100% over 10 minutes, and only buffer B is supplied for 15 minutes to wash the column. Triton X-100 is added to prevent adsorption of AMVRT by the column.

Such gradient elution allows the constituents of AMVRT to be eluted in the order of the α monomer, the αβ heterodimer and the ββ homodimer. Each of these constituents has a reverse transcriptase activity, which is the main activity of AMVRT, but when each constituent is used in gene amplification, the amplification reaction proceeds only in the presence of the αβ heterodimer, not in the presence of the α monomer or the ββ homodimer. Therefore, fractional collection of the effluent in constant amounts followed by determination of the ratio of the α subunit and the β subunit in each fraction affords selection of the αβ heterodimer fractions of interest which contain them in a ratio of about 1. The ratio of the subunits in each fraction can be determined by enzyme immunoassay (EIA) using an antibody specific for each subunit or SDS-PAGE under reductive conditions. The reductive conditions may be created by adding a strong reducing agent such as mercaptoethanol to a sample from each fraction at a concentration of about 5% or by heating a sample from each fraction at 95°C for 5 minutes so that the dimer is compelled to dissociate into monomers.

The determination of the ratio of the α subunit and/or the β subunit using SDS-PAGE may, for example, be effected by staining an electrophoresed gel with a silver stain or the like and numerically measuring the densities of the corresponding bands with a densitometer or the like.

Another form of the AMVRT composition of the present invention, i.e., an AMVRT composition which is substantially a unary composition consisting of the αβ heterodimer is obtainable from the AMVRT composition obtained as described above by using a column packed with an affinity filler having an immobilized antibody against the α subunit and a column packed with an affinity filler having an immobilized antibody against the β subunit.

By the above-mentioned procedure, it is possible to obtain an AMVRT composition which comprises the αβ heterodimer in an amount of at least 80% or an AMVRT composition which is substantially a unary composition consisting of the αβ heterodimer. The AMVRT composition of the present invention thus obtained may be concentrated by ordinary methods such as dialysis and may be stored by ordinary enzyme storing methods.

Now, the present invention will be described in further detail by referring to Examples. However, the present invention is by no means restricted to these specific Examples.

### EXAMPLE 1 PREPARATION OF AMVRT COMPOSITION

500 µl of a commercially available AMVRT preparation (product name; native, 40 U/µl, CHIMERx) was diluted with the following buffer A (4.5 ml).

| Buffer A (final concentrations) | |
|---|---|
| 20 mM | Calcium phosphate (pH 7.2) |
| 2 mM | Dithiothreitol |
| 0.2% | Triton X-100 |
| 10% | Glycerol |

The diluted AMVRT solution was loaded onto a commercially available ion exchange chromatography column (product name; DEAE-5PW, 7.5 mmID×7.5 cm, Tosoh Corporation), and then buffer B shown below was supplied at 4°C, while the effluent was collected at a rate of 1 ml per minute in 60 fractions with the lapse of elution time. The gradient conditions were as follows, and the flow rate of the buffer was 1 ml/min.

| Buffer B (final concentrations) | |
|---|---|
| 20 mM | Calcium phosphate (pH 7.2) |
| 2 mM | Dithiothreitol |
| 0.2% | Triton X-100 |
| 10% | Glycerol |
| 1 M | NaCl |

Gradient conditions
0-15 minutes: Buffer A 100%
15-35 minutes: Gradient from buffer A 100% to buffer A 50% + buffer B 50%
35-45 minutes: Gradient from buffer A 50% +buffer B 50% to buffer B 100%
45-60 minutes: Buffer B 100%

Then, each fraction was subjected to SDS-PAGE under reductive conditions to measure the ratio of the α subunit and the β subunit.

A 5-ml sample from each fraction was mixed with 5 µl of the following SDS-PAGE sample buffer and heated at 95°C for 5 minutes. A 7 µl portion of each sample solution was electrophoresed into a commercially available 10% electrophoresis gel (product name; e-PAGEL E-R10L, 90 mm (W)×73 mm (H)×1 mm (t), ATTO Corporation) at 30 mA. The following buffers were used for the electrophoresis.

| Sample buffer | |
|---|---|
| 0.25 M | Tris-HCl buffer (pH 6.8) |
| 20% | SDS |
| 50% | Glycerol |
| 0.05% | Bromphenol blue |

| Electrophoresis buffer (The volume was adjusted with distilled water to 1 1) | |
|---|---|
| 3.0 g | Tris-HCl |
| 1.0 g | SDS |
| 14.4 g | Glycine |

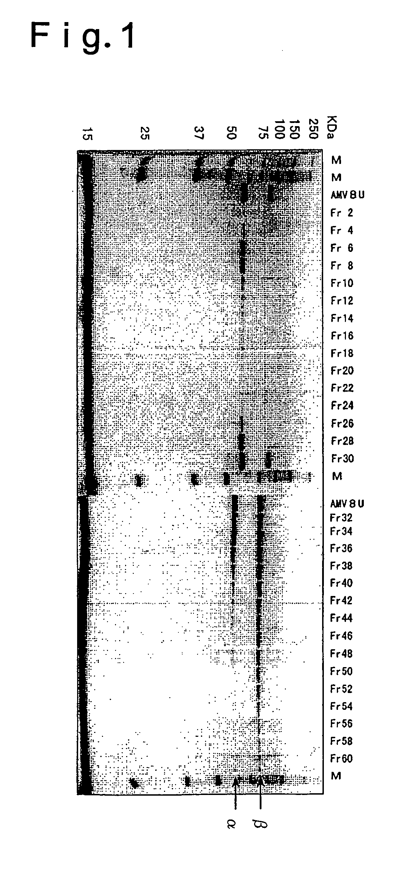

After the electrophoresis, the gel was stained with a commercially available silver stain (product name; Daiichi, Diichi Pure Chemicals Co., Ltd.). The stained gel is shown in Fig. 1. The stained gel was analyzed with a densitometer (product name; Densitograph Lane Analyzer, ATTO Corporation) to calculate the ratios of the α subunit and the β subunit. The results revealed that α monomer composition was eluted in fractions 4-16 and 26-28, the αβ heterodimer composition was eluted in fractions 30-34, and the ββ homodimer composition was eluted in fractions 36-58. The results of the densitometric analysis of the respective fractions (the band density of the α subunit : the band density of the β subunit) were 1:0 in fractions 4-16, 1:0 in fractions 26-28, 55:45 in fractions 30-34, and 16:84 in fractions 36-58. Thus, an α subunit composition, an αβ heterodimer composition and a ββ homodimer composition were obtained in this Example. The αβ composition showed a density ratio of 55:45, thus the result of the densitometric analysis of the αβ composition demonstrated that the composition contained the αβ form in an amount of at least 80%.

### EXAMPLE 2 RT ACTIVITY ASSAY

The AMVRT preparation used in Example 1 was diluted with water to give AMVRT solutions with different concentrations (30 U/µl, 10 U/µl, 3 U/µl, 1 U/µl and 0.1 U/µl). The AMVRT solutions with different concentrations (5 µl each) were incubated with a reaction solution (20 µl) of the following composition at 37°C for 10 minutes.

| Reaction solution (final concentrations) | |
|---|---|
| 50 mM | Tris-HCl buffer (pH 8.3) |
| 40 mM | KCl |
| 8.75 mM | MgCl₂ |
| 10 mM | Dithiothreitol |
| 0.1% | Bovine serum albumin |
| 250 mM | Poly(A) (Pharmacia) |
| 5 mM | Oligo(dT) 30 |
| 1 mM | Thymidine 5'-triphosphate (TTP) sodium salt |
| [methyl-3H] | (Diichi Pure Chemicals Co., Ltd.) |

Then, 133 µl of a liquid mixture (TE 80 µl, Glycogen 3 µl and 7.5 M ammonium acetate 50 µl) and 375 µl of 100% ethanol were added, and the resulting mixtures were left to stand at -20°C for 30 minutes. After 10-minute centrifugation at 4°C and 15000 rpm (radius of gyration 7 cm), the supernatants were removed carefully. The residues were suspended in 100 µl of TE, and the entire suspensions were subjected to scintillation counting to obtain a standard curve. TE is 10 mM Tris-HCl buffer (pH 8.0), 1 mM EDTA.

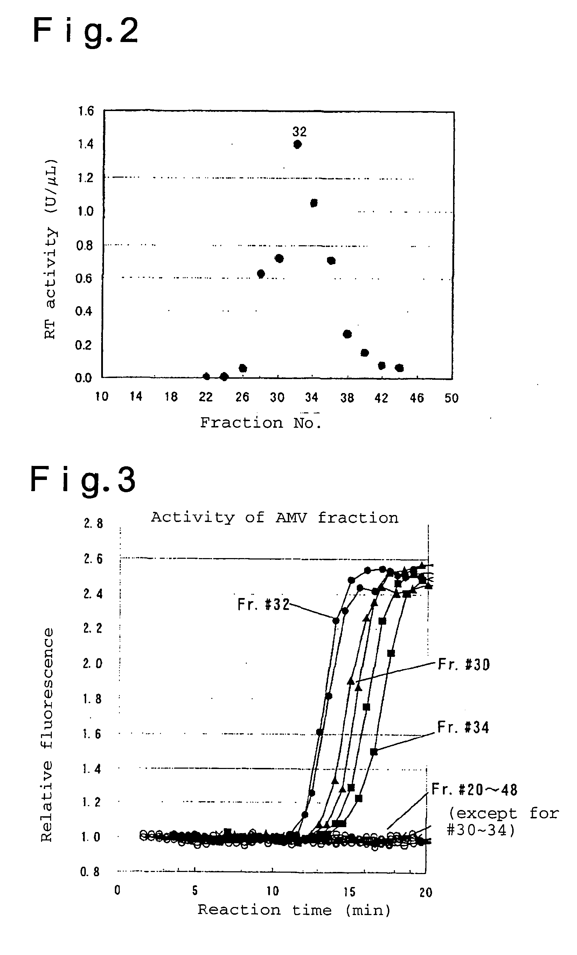

The above-mentioned procedure was followed using 5 µl portions of the fractions obtained in Example 1 instead of the AMVRT solutions, and the resulting suspensions were entirely subjected to scintillation counting. The reverse transcriptase activity of each fraction was obtained from the standard curve obtained above. The results are shown in Fig. 2. Fig. 2 shows that fractions 26-44, i.e., the α monomer composition, the αβ heterodimer composition and the ββ homodimer composition, all had a reverse transcriptase activity.

### EXAMPLE 3 RNA AMPLIFICATION ASSAY

The procedure disclosed in JP-A-2001-353000 was followed to amplify an RNA (hereinafter referred to as mecA-RNA) derived from mecA gene, which is one of the genes of MRSA (Methicillin-Resistant Staphylococcus Aureus) as the target. mecA-RNA used in the present Example was purified from an RNA synthesized by in vitro transcription using a double-stranded DNA containing a nucleotide sequence in mecA as the template.
(1) A standard RNA (2016-mer) containing bases 1 to 2013 (numbered in accordance with Matsuhashi et al., "FEBS Lett. 221, 167-171(1987)") in mecA-RNA encoding a cell-wall-synthesizing protein PBP-2' produced by MRSA, as a sample, was analyzed quantitatively by ultraviolet absorptiometry at 260 nm and diluted with an RNA diluent (10 mM Tris-HCl (pH 8.0), 0.1 mM EDTA, 0.5 U/µl RNase Inhibitor, 5.0 mM DTT) to 1.0X10³ copies/2.5 µl. The diluent alone was used as a control sample (Nega). The initial amount of the RNA was 10³ copies/run.
(2) 23.3 µl portions of a reaction solution of the following composition were dispensed into 0.5 ml PCR tubes (Gene Amp Thin-Walled Reaction Tubes, Perkin Elmer), and 2.5 µl of the above-mentioned RNA sample was added. The composition of the reaction solution (in terms of the concentrations in the reaction system after addition of the enzyme solution)
60.0 mM Tris-HCl buffer (pH 8.6)
13.0 mM Magnesium chloride
90.0 mM Potassium chloride
1.0 mM DTT
0.25 mM each of dATP, dCTP, dGTP and dTTP
3.0 mM each of ATP, CTP and UTP
2.25 mM GTP
3.6 mM ITP
1.0 mM each of a first primer (SEQ ID NO:1) and a second primer (SEQ ID NO:2)
0.16 µ M Scissor oligonucleotide probe (SEQ ID NO:3, an oligonucleotide to cleave the target RNA at a site hybridizable with the first primer which has an aminated 3'-end)
25 nM Oligonucleotide labeled with a fluorescent intercalative dye (SEQ ID NO:4, which has the fluorescent intercalative dye between "G" at position 6 from the 5' end and "A" at position 7 and having a hydroxyl group modified with glycolic acid at the 3' end, Patent No.3189000)
39 U Ribonuclease inhibitor (Takara Shuzo Co., Ltd.)
15.0% DMSO
Distilled water for volume adjustment
(3) The resulting reaction solutions were incubated at 41°C for 5 minutes, and 4.2 µl of enzyme solutions of the following composition containing 5 µl of the AMVRT fractions obtained in Example 1 per 30 µl was added after preincubation at 41°C for 2 minutes.

| The composition of the enzyme solution (in terms of the final concentrations in the reaction) | |
|---|---|
| 1.7% | Sorbitol |
| 142 U | T7 RNA polymerase (GIBCO) |
| 3 µg | Bovine serum albumin |

### Distilled water for volume adjustment

The relative increases in the fluorescence from the reaction solutions in the PCR tubes (fluorescence intensity at a certain time ÷ background fluorescence intensity) were monitored at an excitation wavelength of 470 nm and an emission wavelength of 510 nm with an instrument capable of measuring the fluorescent intensities of the reaction solutions in the tubes thermostatically (JP-A-2000-214090) from the addition of the enzyme solution at 0 minute while the reaction solutions were maintained at 41°C. The results are shown in Fig. 3.

The increase in the fluorescent intensity only in the presence of fractions 30-34 shown in Fig. 3 indicates only the αβ heterodimer composition, which is present in these fractions, can set off the RNA amplification, i.e., has at least a ribonuclease H activity as well as a reverse transcriptase activity. -In contrast, the RNA amplification did not occur in the presence of the other fractions.

### EXAMPLE 4 CONCENTRATION AND RNA AMPLIFICATION ASSAY

The fractions containing the αβ heterodimer composition obtained in Example 1 (fractions 30-34) were concentrated in the following two methods.

In the first method, fractions 30-34 were combined, and 1.3 ml of the resulting mixture was dialyzed in a dialysis tube (SPECTRA/POR, Spectrum Medical Industry Corporation) against 500 mL of an external solution (20 mM calcium phosphate (pH 7.2), 2 mM dithiothreitol, 0.2% Triton X-100) at 4°C for 3 hours, and after renewal of the external solution for another 10 hours. The fraction mixture dialyzate was concentrated to 25 µl with a concentrator (product name; Centricon, Millipore). After the concentration, glycerol was added to a final concentration of 50% as a preservative. The resulting solution containing the αβ heterodimer composition was designated as concentrate 1.

In the second method, 1.3 ml of the above-mentioned fraction mixture was dialyzed in a dialysis tube (SPECTRA/POR, Spectrum Medical Industry Corporation) against 500 mL of an external solution (2 mM calcium phosphate (pH 7.2), 0.2 mM dithiothreitol, 0.02% Triton X-100, 0.01 M trehalose) at 4°C for 3 hours, and after renewal of the external solution for another 10 hours. The fraction mixture dialyzate was concentrated to 130 µl with a concentrator (product name; Centricon, Millipore). The resulting solution containing the αβ heterodimer composition was designated as concentrate 2.

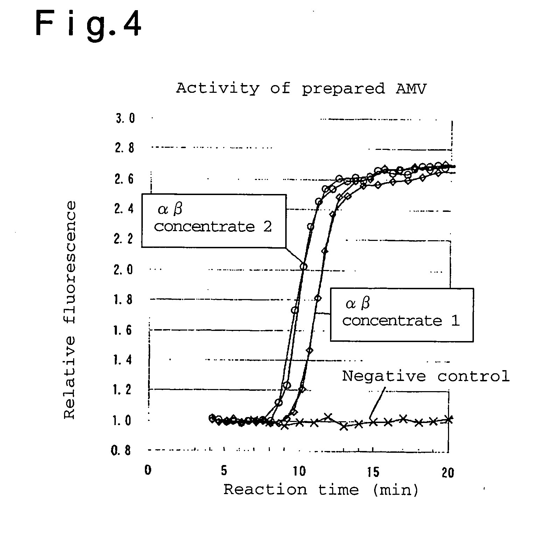

Concentrates 1 and 2 thus obtained were used for amplification of mecA-RNA in the same manner as in Example 3 provided that instead of the AMVRT solution used in Example 3, 0.25 µl of concentrate 1 or 2 was used per 30 µl of the reaction solution. The results are shown in Fig. 4. Fig. 4 demonstrates that the RNA was amplified in the presence of concentrate 1 or 2.

The measure (unit) for the amount of AMVRT is conventionally based on its main activity as a reverse transcriptase, i.e., an RNA-dependent DNA polymerase activity. Therefore, even if a certain amount of conventional ternary AMVRT is measured out' in units, the actual contribution of the AMVRT to the above-mentioned gene amplification is not always the same. Besides, the ratio of the three constituents, the α monomer, the αβ heterodimer and the ββ homodimer, is likely to vary depending on the production lot and the distributor of the AMVRT. If such a compositional change affects the ratio of the RNA-dependent DNA polymerase activity, the DNA-dependent DNA polymerase activity or the ribonuclease H activity, even a fixed amount of the enzyme can not give the same amplification efficiency, and its influence on qualitative and quantitative gene analysis using such an amplification reaction is easily predictable.

Therefore, the use of the AMVRT composition of the present invention affords higher amplification efficiency per 1 unit of the AMVRT composition and therefore makes it possible to amplify genes as efficiently as ever even with a smaller amount of AMVRT. At the same time, it is possible to compare the results of qualitative or quantitative analysis obtained by using different lots of AMVRT under the same gene amplification conditions more strictly.

## Claims

1. An avian myeloblastosis virus reverse transcriptase composition which comprises a heterodimer consisting of an α subunit having a molecular weight of 68000 and a β subunit having a molecular weight of 92000 in an amount of at least 80%.

2. The avian myeloblastosis virus reverse transcriptase composition which is a unary composition consisting of a heterodimer consisting of an α subunit having a molecular weight of 68000 and a β subunit having a molecular weight of 92000.

3. A method of preparing a heterodimer consisting of an α subunit having a molecular weight of 68000 and a β subunit having a molecular weight of 92000 from an avian myeloblastosis virus reverse transcriptase solution containing the αβ heterodimer, and the α subunit and/or the homodimer of the β subunit, which comprises subjecting the solution to ion exchange chromatography.

4. The method according to Claim 3, wherein the ion exchange chromatography uses an ion exchanger having diethylaminoethyl groups as ion exchange groups.

5. A reagent for use in amplification of a target RNA or an RNA complementary to the target RNA comprising hybridizing a first DNA primer to the target RNA, elongating the first DNA primer into a DNA-RNA heteroduplex nucleic acid, degrading the target RNA in the DNA-RNA heteroduplex nucleic acid, hybridizing a second DNA primer to the residual DNA, elongating the second DNA primer into a double-stranded DNA, transcribing the double-stranded DNA into the target RNA or an RNA complementary to the target RNA (wherein either the first or second DNA primer has a promoter region), which comprises the avian myeloblastosis virus reverse transcriptase composition according to Claim 1.


SEQUENCE LISTING

<110> TOSOH Corporation

<120> Unary avian myeloblastosis virus reverse transcriptase and its use

<130> H 1401 EP

<160> 4

<210> 1
<211> 28
<212> DNA
<213> Artificial Sequence

<220>
<223> Primer

<400> 1
caactaacta ttgatgctaa agttcaaa 28

<210> 2
<211> 20
<212> DNA
<213> Artificial Sequence

<220>

<223> Primer

<400> 2

ttctttttta tcttcggtta                                                    20

<210> 3

<211> 39

<212> DNA

<213> Artificial Sequence

<220>

<223> Probe

<400> 3

gttagttgaa tatctttgcc atcttttttc tttttctct                               39

<210> 4

<211> 20

<212> DNA

<213> Artificial Sequence

<220>

<223> Oligonucleotide labeled with a fluorescent intercalative dye

<400> 4

tgtttgaggg tggatagcag                                                    20